# FASCICULE DE BREVET EUROPEEN

(11) **EP 0 864 305 B1**
(45) Date de publication et mention de la délivrance du brevet: **07.08.2002**
(21) Numéro de dépôt: 98420041.0
(22) Date de dépôt: 05.03.1998
(51) Int. Cl.: A61F 2/42

(54) **Phrothèse de cheville**
Knöchelprothese
Ankle prosthesis

(30) Priorité: 10.03.1997 FR 9703040
(43) Date de publication de la demande: 16.09.1998
(73) Titulaire: TORNIER SA, 38330 Saint-Ismier (FR)
(72) Inventeur: Bonnin, Michel, 69340 Francheville (FR); Colombier, Jean-Alain, 31130 Balma (FR); Judet, Thierry, 92410 Ville D'Avray (FR); Tornier, Alain, 38330 Saint Ismier (FR)
(74) Mandataire: Myon, Gérard Jean-Pierre

(56) Documents cités:
- DE-A- 2 401 481
- FR-A- 2 370 465
- FR-A- 2 684 291
- FR-A- 2 700 462
- FR-A- 2 724 108
- US-A- 3 975 778
- US-A- 4 232 404

## Description

La présente invention concerne les prothèses de cheville.

De nombreuses prothèses de cheville ont été proposées, qui comprennent une partie tibiale et une partie astragalienne munies chacune d'une surface articulaire, ces surfaces articulaires etant conformées de façon a coopérer ensemble pour fournir l'articulation souhaitée encre le pied et la jambe. Diverses formes de surfaces articulaires ont été envisagées, les unes sphériques, d'autres constituées par des surfaces de révolution autour d'un axe sensiblement perpendiculaire à l'axe tibial, et n'autorisant un mouvement de rotation qu'autour de ce seul axe. De plus, l'une ou l'autre des parties tibiale et astragalienne peut comporter une interface plane autorisant un glissement entre deux pièces, l'une fixée à l'os et l'autre délimitant la surface articulaire.

Des prothèses de cheville selon le préambule de la revendication 1 de la présente invention sont divulguées dans les documents US 3 957 778 et FR 2 370 465.

On constate cependant que pour les diverses prothèses actuellement utilisées, le mouvement d'articulation obtenu ne correspond pas au mouvement naturel d'articulation de la cheville, ce qui se traduit par une inadéquation entre le mouvement autorisé par les surfaces articulaires et le mouvement que les différents ligaments tendent à imposer au pied. Cette inadéquation se traduit par l'apparition de contraintes et de sollicitations au niveau de la prothèse, qui peuvent provoquer une usure, une perte de mobilité ou même un descellement de cette dernière. Il en résulte par ailleurs une gêne et des douleurs désagréables pour le patient.

Le but de cette invention est en conséquence de proposer une prothèse de cheville qui permette d'améliorer cette situation et d'obtenir une cinématique d'articulation qui se rapproche davantage de celle d'une cheville normale.

A cet effet, l'invention a pour objet une prothèse de cheville, comprenant une partie tibiale et une partie astragalienne, munies chacune de moyens de fixation, respectivement sur l'extrémité inférieure d'un tibia et sur l'astragale, et délimitant chacune une surface articulaire, les deux surfaces articulaires étant conformées pour coopérer ensemble et autoriser un déplacement relatif entre les deux parties tibiale et astragalienne autour d'au moins un axe, caractérisée en ce que, prenant comme direction de référence la direction de l'axe du tibia sur lequel la partie tibiale est destinée à être fixée, vues en projection sur un plan sensiblement perpendiculaire à cette direction de référence, les surfaces articulaires sont incurvées, avec une concavité dirigée vers la malléole interne du pied sur lequel la partie astragalienne est destinée à être fixée.

Suivant d'autres caractéristiques :
- les surfaces articulaires comportent chacune une surface centrale d'appui et deux surfaces latérales de butée, un jeu étant prévu entre les surfaces latérales de butée des deux surfaces articulaires, autorisant un débattement latéral limité entre ces deux surfaces articulaires ;
- le jeu entre les surfaces latérales de butée varie sur la longueur des surfaces articulaires ;
- le jeu entre les surfaces latérales de butée est plus important dans la zone arrière d'une articulation équipée de la prothèse que dans sa zone avant ;
- le jeu entre les surfaces latérales de butée correspond à un débattement angulaire inférieur à 5° de part et d'autre d'une position médiane ;
- les surfaces articulaires comportent chacune une surface centrale et deux surfaces latérales d'appui, ces deux dernières étant situées à des niveaux différents de celui de la surface centrale d'appui, les surfaces latérales de butée étant constituées par des zones de raccordement entre les surfaces centrale et latérales d'appui ;
- chaque surface articulaire peut être définie comme étant engendrée par la rotation d'une ligne directrice plane comportant au moins un arc de cercle, autour d'au moins un axe contenu dans son plan et orienté obliquement par rapport au rayon moyen ou médian dudit arc de cercle ;
- l'axe autour duquel on fait tourner la directrice, celle-ci étant supposée contenue dans un plan sensiblement vertical, monte en direction de la malléole interne du pied sur lequel la partie astragalienne est destinée à être fixée, en étant orienté vers le haut par rapport au rayon moyen ou médian de cette directrice ;
- la directrice est formée de trois arcs de cercle, un arc central et deux arcs latéraux, reliés par deux courbes présentant chacune un point d'inflexion ;
- la partie astragalienne de la prothèse comporte une partie articulaire latérale à double courbure, convexe suivant une direction avant-arrière et concave suivant une direction verticale, adaptée pour coopérer avec la partie adjacente de l'extrémité inférieure du péroné.

L'invention va être décrite plus en détail ci-dessous en se référant aux dessins annexés, donnés uniquement à titre d'exemples et sur lesquels :
- la figure 1 est une vue de devant ou frontale avec coupe partielle d'une prothèse selon l'invention, pour un pied gauche ;
- les figures 2 à 4 sont, respectivement des vues de dessus, en élévation latérale et de dessous de la pièce astragalienne ;
- la figure 5 est un schéma précisant le profil et le mode de génération des surfaces articulaires ; et
- la figure 6 est une vue schématique en plan d'une variante.

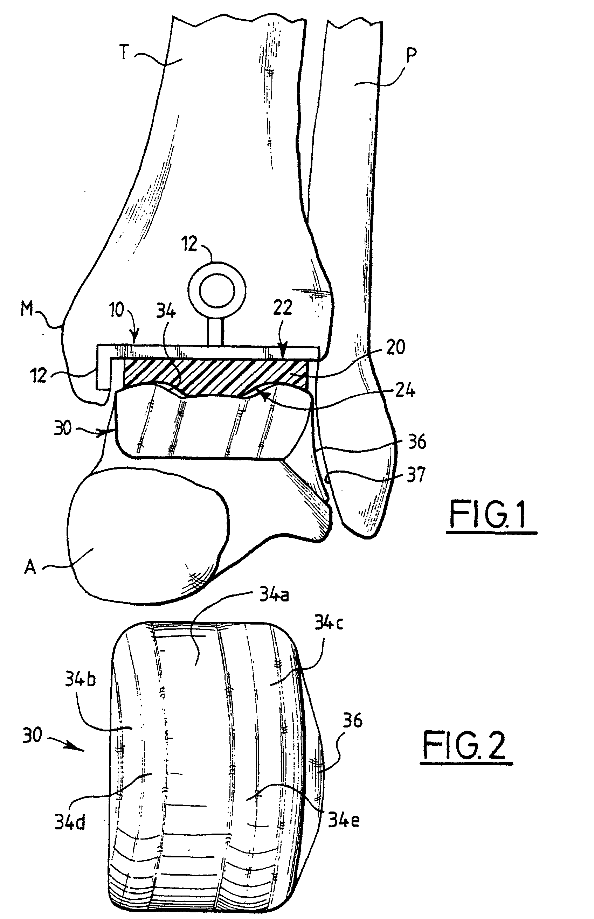

On voit sur la figure 1 les extrémités inférieures d'un tibia T et d'un péroné P, ainsi que l'astragale A située au voisinage des extrémités de ces deux os.

La prothèse selon l'invention comprend une plaque 10 fixée à l'extrémité inférieure du tibia, cette extrémité étant bien entendu convenablement préparée. Dans l'exemple représenté, la plaque tibiale 10 est fixée dans l'os au moyen d'un rail d'ancrage creux 12 mais tout autre moyen convenable de fixation pourrait être utilisé. Cette plaque comporte un rebord 12, sur son côté dirigé vers la malléole interne M. Elle peut être réalisée en tout matériau biocompatible, tel que : acier au chrome, titane, céramique ou autre.

Un patin 20 réalisé en un matériau à faible coefficient de frottement, par exemple en polyéthylène haute densité comporte une surface plane 22 en appui sur la plaque 10, et délimite sur sa face opposée une surface articulaire 24. Afin de limiter les débattements entre la plaque 10 et le patin 20, la plaque peut comporter un plot reçu dans un logement ménagé dans la face plane du patin, les dimensions de ce logement déterminant la course du déplacement relatif autorisé entre les deux pièces. De tels moyens, connus dans la technique n'ont pas été représentés.

L'ensemble formé par la plaque 10 et le patin 20 constitue la partie tibiale de la prothèse.

La partie astragalienne est constituée par un bloc 30 en acier au chrome, titane ou céramique, par exemple, qui est fixé dans la partie supérieure de l'astragale par un plot creux 32 et une nervure 33 ou par tout autre moyen convenable, et qui délimite une surface articulaire supérieure 34 destinée à coopérer avec la surface articulaire conjuguée 24 délimitée par le patin 20.

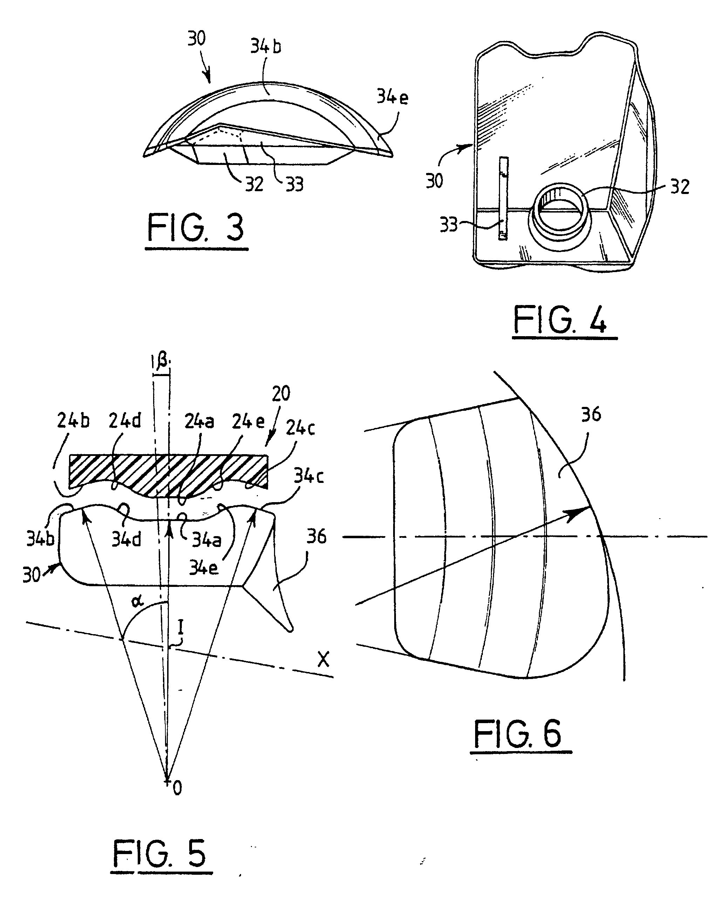

Selon l'invention et comme représenté aux dessins (Figs. 1 et 5), la surface articulaire astragalienne comporte une surface centrale d'appui 34a à double courbure frontale et sagittale. Deux autres surfaces latérales d'appui 34b, 34c sont également prévues, qui présentent également une double courbure. Les deux surfaces latérales d'appui ne sont pas situées au même niveau que la surface centrale et les zones de liaison 34d et 34e entre la zone centrale et les zones latérales constituent des surfaces de butée pour la surface articulaire tibiale conjuguée.

Dans le mode de réalisation représenté (Fig. 5), la surface articulaire astragalienne 34 est engendrée par une courbe directrice, contenue dans un plan et comportant une partie centrale et deux parties latérales formées par des arcs de cercles concentriques, reliées par des portions de courbes présentant un point d'inflexion. Ces différentes portions de la directrice sont désignées par les mêmes références 34a à 34e que les surfaces correspondantes. A titre d'exemple, les rayons de la partie centrale 34a et des parties latérales 34b, 34c peuvent être respectivement d'environ 35 mm et 40 mm.

Pour engendrer l'ensemble de la surface articulaire astragalienne, on fait tourner la directrice autour d'un axe X qui est contenu dans le plan de cette directrice et qui est orienté obliquement par rapport au rayon moyen ou rayon médian de la partie centrale 34a. De préférence, l'angle α que forme cet axe avec le rayon médian est compris entre 70 et 85° et la distance entre le point I d'intersection de cet axe avec le rayon médian et la partie centrale de la directrice est de l'ordre de 10 à 24 mm.

Ce mode de génération de la surface articulaire se traduit par le fait que vue en plan ou en projection sur un plan sensiblement perpendiculaire à l'axe du tibia (Fig. 2), la surface articulaire a une forme incurvée, avec sa concavité dirigée vers la malléole interne M ou encore vers l'intérieur du pied du patient susceptible de porter une telle prothèse.

La pièce 30 comporte de plus une partie latérale 36 délimitant une surface articulaire coopérant avec la partie adjacente 37 de l'extrémité inférieure du péroné. Suivant une caractéristique importante, cette surface est convexe dans le sens avant-arrière et concave dans le sens vertical. Vue en projection sur un plan sensiblement horizontal ou perpendiculaire à l'axe du tibia, elle peut être sensiblement concentrique à la surface articulaire 34 (Fig. 2) ou au contraire être centrée sur un point décalé vers l'avant par rapport au centre de courbure de la surface 34 (Fig. 6).

La surface articulaire tibiale 24 délimitée par la face inférieure du patin 20 présente un profil analogue à celui de la surface articulaire astragalienne, avec des formes complémentaires, à savoir concaves alors que la surface articulaire astragalienne présente une forme convexe. On retrouve donc dans cette surface articulaire des parties centrale 24a et latérales 24b, 24c d'appui, coopérant avec les parties correspondantes de l'autre surface articulaire, la partie centrale 24a étant cependant de préférence moins large que l'autre partie centrale 34a, de façon à ménager un jeu latéral déterminé entre les surfaces de butée 24d, 24e, 34d, 34e, constituées par les zones de raccordement ou de transition entre les surfaces centrales et les surfaces latérales d'appui.

Ce jeu latéral autorise, par exemple, de part et d'autre d'une position moyenne, un débattement angulaire ± β de ± 2° autour du centre instantané de rotation qui est constitué par le centre O desdites surfaces d'appui latérales. L'angle β peut être compris entre 0 et 5°.

Une telle prothèse procure une cinématique qui est très proche de celle de l'articulation naturelle de la cheville et assure donc une bonne adequation entre le mouvement d'articulation procuré par les surfaces articulaires et les forces exercées par les ligaments. De plus, on réalise une bonne congruence entre les surfaces articulaires et un positionnement très favorables des centres instantanés de rotation dans tes différentes positions de la cheville.

On peut ajouter que dans les cas où une prothèse de cheville doit être envisagée, il est fréquent que le calcanéum soit usé, ce qui se traduit chez le patient par un blocage du mouvement entre le talon et l'astragale. La prothèse selon l'invention, grâce au jeu latéral limité qui est prévu, permet de compenser dans une large mesure cet inconvénient.

A titre de variante, on peut envisager de prévoir pour ce jeu angulaire latéral une valeur plus importante à l'arrière de l'articulation que dans sa partie avant. C'est ainsi que ce jeu angulaire qui peut être de ± 2° dans la partie avant, peut augmenter jusqu'à ± 4 ou 5° dans la partie arrière.

De nombreuses variantes peuvent par ailleurs être apportées à cette prothèse, concernant notamment les modes d'ancrage sur le tibia et sur l'astragale, ainsi que la forme et la réalisation des pièces la constituant.

Dans la mesure où les surfaces articulaires présentent la courbure souhaitée, avec une concavité dirigée vers l'intérieur, elles peuvent être réalisées suivant des profils différents de celui représenté. Par exemple, dans une première partie s'étendant de l'extrémité arrière jusqu'à deux tiers ou trois quarts de la longueur des surfaces articulaires, ces surfaces articulaires peuvent être engendrées comme décrit ci-dessus par rotation de la directrice autour de l'axe X, tandis que dans la zone avant restante, la surface peut être engendrée par rotation de cette même directrice autour d'un deuxième axe plus proche de la directrice et parallèle ou non à l'axe X. Dans ce cas, la courbure, en projection sur un plan sensiblement horizontal, est plus importante dans la partie avant que dans la partie arrière des surfaces articulaires. La position et l'inclinaison de ce deuxième axe sont alors choisies de façon à obtenir la courbure souhaitée et une transition harmonieuse avec la première partie de la surface.

## Revendications

1. Prothèse de cheville, comprenant une partie tibiale (10, 20) et une partie astragalienne (30), munies chacune de moyens de fixation, respectivement sur l'extrémité inférieure d'un tibia (T) et sur l'astragale (A), et délimitant chacune une surface articulaire (24, 34), les deux surfaces articulaires étant conformées pour coopérer ensemble et autoriser un déplacement relatif entre les deux parties tibiale et astragalienne autour d'au moins un axe, **caractérisée en ce que**, prenant comme direction de référence la direction de l'axe du tibia (T) sur lequel la partie tibiale est destinée à être fixée, vues en projection sur un plan sensiblement perpendiculaire à cette direction de référence, les surfaces articulaires (24, 34) sont incurvées, avec une concavité dirigée vers la malléole interne (M) du pied sur lequel la partie astragalienne est destinée à être fixée.

2. Prothèse de cheville selon la revendication 1, **caractérisée en ce que** les surfaces articulaires (24, 34) comportent chacune une surface centrale d'appui (24a, 34a) et deux surfaces latérales de butée (24d, 24e, 34d, 34e).

3. Prothèse de cheville selon la revendication 1, **caractérisée en ce qu'**un jeu est prévu entre les surfaces latérales de butée des deux surfaces articulaires, autorisant un débattement latéral limité entre ces deux surfaces articulaires.

4. Prothèse de cheville selon la revendication 3, **caractérisée en ce que** le jeu entre les surfaces latérales de butée varie sur la longueur des surfaces articulaires.

5. Prothèse de cheville selon la revendication 4, **caractérisée en ce que** le jeu entre les surfaces latérales de butée est plus important dans la zone arrière d'une articulation équipée de la prothèse que dans sa zone avant.

6. Prothèse de cheville selon l'une quelconque des revendications 1 à 5, **caractérisée en ce que** les surfaces articulaires (24, 34) comportent chacune une surface centrale (24a, 34a) et deux surfaces latérales (24b, 24c, 34b, 34c) d'appui, ces deux dernières étant situées à des niveaux différents de celui de la surface centrale d'appui, les surfaces latérales de butée (24d, 24e, 34d, 34e) étant constituées par des zones de raccordement entre les surfaces centrale et latérales d'appui.

7. Prothèse de cheville selon l'une quelconque des revendications 1 à 6, **caractérisée en ce que** chaque surface articulaire (24, 34) peut être définie comme étant engendrée par la rotation d'une ligne directrice plane comportant au moins un arc de cercle, autour d'au moins un axe (X) contenu dans son plan et orienté obliquement par rapport au rayon moyen ou médian dudit arc de cercle.

8. Prothèse de cheville selon les revendications 3 et 7, **caractérisée en ce que** le jeu entre les surfaces latérales de butée correspond à un débattement angulaire (β) inférieur à 5°, de part et d'autre d'une position médiane.

9. Prothèse de cheville selon la revendication 7, **caractérisée en ce que** l'axe (X) autour duquel on fait tourner la directrice, celle-ci étant supposée contenue dans un plan sensiblement vertical, monte en direction de la malléole interne (M) du pied sur lequel la partie astragalienne est destinée à être fixée en étant orienté vers le haut par rapport au rayon moyen ou médian de cette directrice.

10. Prothèse de cheville selon la revendication 9, **caractérisée en ce que** ledit axe (X) forme un angle (α) compris entre 70 et 85° avec le rayon moyen ou médian de la directrice.

11. Prothèse de cheville selon l'une quelconque des revendications 7 à 10, **caractérisée en ce que** la distance entre le point d'intersection de l'axe (X) de la surface articulaire (24, 34) avec le rayon moyen ou médian de cette dernière, et la partie centrale (24a, 34a) de la directrice est comprise entre 10 et 24 mm.

12. Prothèse de cheville selon l'une des revendications 7 à 11, **caractérisée en ce que** ladite directrice est formée de trois arcs de cercle, un arc central (24a ; 34a) et deux arcs latéraux (24b, 24c ; 34b, 34c) reliés par deux courbes (24d, 24e ; 34d, 34e) présentant chacune un point d'inflexion.

13. Prothèse de cheville selon l'une quelconque des revendications précédentes, **caractérisée en ce que** vue en projection sur le plan perpendiculaire à ladite direction de référence, la courbure des surfaces articulaires est plus importante dans la partie avant d'une articulation équipée de la prothèse que dans sa partie arrière.

14. Prothèse de cheville selon l'une quelconque des revendications précédentes, **caractérisée en ce que** la partie astragalienne (30) de la prothèse comporte une partie latérale (36) délimitant une surface articulaire à double courbure, convexe suivant une direction avant-arrière et concave suivant une direction verticale, adaptée pour coopérer avec la partie adjacente (37) de l'extrémité inférieure du péroné.

## Claims

1. Ankle prosthesis, comprising a tibial part (10, 20) and an astragalar part (30), each of which is provided with means for securing, respectively on the lower end of a tibia (T) and on the astragalus (A), and each delimiting an articular surface (24, 34), the two articular surfaces being formed such as to co-operate with one another and to permit relative displacement between the two tibial and astragalar parts around at least one axis, **characterised in that**, taking as the reference direction the direction of the axis of the tibia (T) on which the tibial part is designed to be secured, seen in projection on a plane which is substantially perpendicular to this reference direction, the articular surfaces (24, 34) are curved, with concavity which faces the inner malleolus (M) of the foot on which the astragalar part is designed to be secured.

2. Ankle prosthesis according to claim 1, **characterised in that** the articular surfaces (24, 34) each comprise a central support surface (24a, 34a) and two lateral stop surfaces (24d, 24e, 34d, 34e).

3. Ankle prosthesis according to claim 1, **characterised in that** play is provided between the lateral stop surfaces of the two articular surfaces, thus permitting limited lateral clearance between these two articular surfaces.

4. Ankle prosthesis according to claim 3, **characterised in that** the play between the lateral stop surfaces varies along the length of the articular surfaces.

5. Ankle prosthesis according to claim 4, **characterised in that** the play between the lateral stop surfaces is greater in the rear area of an articulation equipped with the prosthesis than in its front area.

6. Ankle prosthesis according to any one of claims 1 to 5, **characterised in that** the articular surfaces (24, 34) each comprise a central surface (24a, 34a) and two lateral support surfaces (24b, 24c, 34b, 34c), these two latter being situated at levels which are different from that of the central support surface, the lateral stop surfaces (24d, 24e, 34d, 34e) consisting of areas of connection between the central and lateral support surfaces.

7. Ankle prosthesis according to any one of claims 1 to 6, **characterised in that** each articular surface (24, 34) can be defined as being generated by the rotation of a flat guide line which comprises at least one arc of a circle, around at least one axis (X) which is contained in its plane and is oriented obliquely relative to the median or mean radius of the said arc of a circle.

8. Ankle prosthesis according to claim 3 and claim 7, **characterised in that** the play between the lateral stop surfaces corresponds to an angular clearance (β) of less than 5° on both sides of a median position.

9. Ankle prosthesis according to claim 7, **characterised in that** the axis (X) around which the directrix is rotated, the latter being assumed to be contained in a substantially vertical plane, rises in the direction of the inner malleolus (M) of the foot on which the astragalar part is designed to be secured whilst being oriented upwards relative to the median or mean radius of this directrix.

10. Ankle prosthesis according to claim 9, **characterised in that** the said axis (X) forms an angle (α) of between 70 and 85° with the median or mean radius of the directrix.

11. Ankle prosthesis according to any one of claims 7 to 10, **characterised in that** the distance between the point of intersection of the axis (X) of the articular surface (24, 34) with the median or mean surface of the latter and the central part (24a, 34a) of the directrix is between 10 and 24 mm.

12. Ankle prosthesis according to any one of claims 7 to 11, **characterised in that** the said directrix is formed by three arcs of a circle, a central arc (24a; 34a) and two lateral arcs (24b, 24c; 34b, 34c) which are connected by two curves (24d, 24e; 34d, 34e) each of which has a point of inflexion.

13. Ankle prosthesis according to any one of the preceding claims, **characterised in that** seen in projection on the plane which is perpendicular to the said reference direction, the curvature of the articular surfaces is greater in the front part of an articulation equipped with the prosthesis than in its rear part.

14. Ankle prosthesis according to any one of the preceding claims, **characterised in that** the astragalar part (30) of the prosthesis comprises a lateral part (36) which delimits an articular surface with a double curvature, which is convex in a front-rear direction and concave in a vertical direction, and is designed to co-operate with the adjacent part (37) of the lower end of the fibula.

## Patentansprüche

1. Fußgelenksprothese mit einem Schienbeinteil (10, 20) und einem Sprungbeinteil (30), die jeweils mit Mitteln zur Befestigung auf dem unteren Ende eines Schienbeins (T) beziehungsweise auf dem Sprungbein (A) versehen sind und jeweils eine Gelenkfläche (24, 34) begrenzen, wobei die zwei Gelenkflächen so geformt sind, dass sie zusammenwirken und eine Relativbewegung zwischen den zwei Schienbein- und Sprungbeinteilen um zumindest eine Achse gestatten, **dadurch gekennzeichnet, dass**, wenn als Bezugsrichtung die Richtung der Achse des Schienbeins (T) angenommen wird, auf der der Schienbeinteil befestigt werden soll, die Gelenkflächen (24, 34), gesehen in Projektion auf eine zu dieser Bezugsrichtung im wesentlichen senkrechten Ebene, gekrümmt sind, mit einer Konkavität, die zum inneren Knöchel (M) des Fußes gerichtet ist, an dem der Sprungbeinteil befestigt werden soll.

2. Fußgelenksprothese nach Anspruch 1, **dadurch gekennzeichnet, dass** die Gelenkflächen (24, 34) jeweils eine zentrale Auflagefläche (24a, 34a) und zwei seitliche Anschlagflächen (24d, 24e, 34d, 34e) umfassen.

3. Fußgelenksprothese nach Anspruch 1, **dadurch gekennzeichnet, dass** zwischen den seitlichen Anschlagflächen der zwei Gelenkflächen ein Spiel vorgesehen ist, das eine begrenzte seitliche Verschiebbarkeit zwischen diesen zwei Gelenkflächen zulässt.

4. Fußgelenksprothese nach Anspruch 3, **dadurch gekennzeichnet, dass** das Spiel zwischen den seitlichen Anschlagflächen über die Länge der Gelenkflächen variiert.

5. Fußgelenksprothese nach Anspruch 4, **dadurch gekennzeichnet, dass** das Spiel zwischen den seitlichen Anschlagflächen im hinteren Bereich eines mit der Prothese ausgestatteten Gelenks größer ist als in seinem vorderen Bereich.

6. Fußgelenksprothese nach einem der Ansprüche 1 bis 5, **dadurch gekennzeichnet, dass** die Gelenkflächen (24, 34) jeweils eine zentrale Auflagefläche (24a, 34a) und zwei seitliche Auflageflächen (24b, 24c, 34b, 34c) aufweisen, wobei diese letzteren auf Höhen sitzen, die verschieden zu derjenigen der zentralen Auflagefläche sind, wobei die seitlichen Anschlagflächen (24d, 24e, 34d, 34e) von zwei Verbindungsbereichen zwischen der zentralen und den seitlichen Auflageflächen gebildet werden.

7. Fußgelenksprothese nach einem der Ansprüche 1 bis 6, **dadurch gekennzeichnet, dass** jede Gelenkfläche (24, 34) definiert werden kann, als wenn sie durch die Rotation einer ebenen Leitlinie, die zumindest einen Kreisbogen umfasst, um zumindest eine in ihrer Ebene enthaltene und zum Mittel- oder Medianstrahl des Kreisbogens geneigte Achse (X) erzeugt wird.

8. Fußgelenksprothese nach den Ansprüchen 3 und 7, **dadurch gekennzeichnet, dass** das Spiel zwischen den seitlichen Anschlagflächen einem Winkelausschlag (β) von kleiner als 5° auf beiden Seiten einer Mittelposition entspricht.

9. Fußgelenksprothese nach Anspruch 7, **dadurch gekennzeichnet, dass** die Achse (X), um die man die Leitlinie sich drehen lässt, von der man annimmt, dass sie in einer im wesentlichen vertikalen Ebene enthalten ist, in Richtung des inneren Knöchels (M) des Fusses ansteigt, an dem das Sprungbeinteil befestigt werden soll, wobei sie bezüglich des Mittel- oder Medianstrahls dieser Leitlinie nach oben gerichtet ist.

10. Fußgelenksprothese nach Anspruch 9, **dadurch gekennzeichnet, dass** die Achse (X) mit dem Mittel- oder Medianstrahl der Leitlinie einen Winkel (α) bildet, der zwischen 70 und 85° liegt.

11. Fußgelenksprothese nach einem der Ansprüche 7 bis 10, **dadurch gekennzeichnet, dass** der Abstand zwischen dem Schnittpunkt der Achse (X) der Gelenkfläche (24, 34) mit dem Mittel- oder Medianstrahl dieser letzteren und dem zentralen Teil (24a, 34a) der Leitlinie zwischen 10 und 24 mm liegt.

12. Fußgelenksprothese nach einem der Ansprüche 7 bis 11, **dadurch gekennzeichnet, dass** die Leitlinie von drei Kreisbögen gebildet wird, einem zentralen Kreisbogen (24a; 34a) und zwei seitlichen Kreisbögen (24b, 24c; 34b, 34c), die durch zwei Kurven (24d, 24e; 34d, 34e) verbunden sind, die jeweils einen Wendepunkt aufweisen.

13. Fußgelenksprothese nach einem der vorhergehenden Ansprüche, **dadurch gekennzeichnet, dass**, gesehen in Projektion auf die Ebene senkrecht zur Bezugsrichtung, die Krümmung der Gelenkflächen in dem Teil vor einem mit der Prothese ausgestatteten Gelenk größer ist, als in seinem hinteren Teil.

14. Fußgelenksprothese nach einem der vorhergehenden Ansprüche, **dadurch gekennzeichnet, dass** der Sprungbeinteil (30) der Prothese einen seitlichen Teil (36) umfasst, der eine Gelenkfläche mit doppelter Krümmung begrenzt, konvex längs einer Richtung von vorn nach hinten und konkav längs einer vertikalen Richtung, die dafür ausgelegt ist, mit dem angrenzenden Teil (37) des unteren Endes des Wadenbeins zusammenzuwirken.
